# EUROPEAN PATENT APPLICATION

(11) **EP 3 939 505 A1**
(43) Date of publication of application: **19.01.2022**
(21) Application number: 20185600.2
(22) Date of filing: 14.07.2020
(51) Int. Cl.: A61B 5/1455, A61B 5/1486, A61B 5/1491, A61B 5/145, A61B 5/00

(54) **DEVICE FOR OBTAINING AN INDICATOR OF A MICROCIRCULATORY CONDITION**

(71) Applicant: SenTec AG, 4106 Therwil (CH); Erasmus University Medical Center Rotterdam, 3015 CE Rotterdam (NL)
(72) Inventor: Van Weteringen, Willem, 3011 TT Rotterdam (NL); Goos, Tomas Gijsbertus, 2628 RN Delft (NL); Hayoz, Josef, 4133 Pratteln (CH)
(74) Representative: Hepp Wenger Ryffel AG

(57) **Abstract**

The invention relates to a device (1) and a method for obtaining an indicator of the microcirculatory condition of a patient, the device (1) comprises at least one sensor (2) for measuring data indicative of an arterial blood oxygen level, at least one sensor (3) for measuring data indicative of a tissue oxygen level and a control unit (4) for determining a measure of microcirculation, in particular changes in tissue perfusion on the basis of the tissue oxygen level and the arterial blood oxygen level.

## Description

The invention relates to a device and a method for obtaining an indicator of a microcirculatory condition of a patient.

Several clinical situations, such as sepsis, haemorrhage and cardiac arrest lead to a decrease of tissue perfusion. The small blood vessels and capillaries provide for the microcirculation, which may be affected. This microcirculatory impairment may lead to a dysfunction or even failure of organs which worsens the patient's prognosis.

When bacteria enter the bloodstream and cause a systemic infection to which the body responds, this is called sepsis. Sepsis is one of the largest causes of morbidity in the Intensive Care Unit (ICU) in neonates, children and adults. The incidence of sepsis is approximately 25% in high-level neonatal ICUs. Multiple factors including prematurity, immunological deficiencies and multiple entry points such as lines/intravascular catheters often lead to infiltration of organisms into the bloodstream.

Pathological changes due to sepsis might be monitored by detecting EEG encephalopathy, fever, hypothermia, core/peripheral temperature difference, decreased temperature variability, tachypnea, apnea, increased respiratory rate variability, cardio-respiratory uncoupling, decreased heart rate variability, transient decelerations, decreased blood pressure, decreased blood pressure variability and decreased perfusion and tissue oxygenation. The golden standard remains a positive blood culture in which pathological bacteria are cultured. By the time the clinical manifestation of sepsis becomes eminent and a blood culture is drawn, the clinical condition of the patient may have already deteriorated severely. Mortality and morbidity are significant despite prompt start of an antibiotic treatment. As a consequence of the time delay between drawing blood for a culture to the resulting bacterial growth, blood cultures in practice serve to confirm the presence of bacteria and specify the type of bacteria for adjustment of treatment. When sepsis is suspected on the basis of clinical signs, therapy is therefore started empirically. Detecting sepsis in an early stage or even confirming it when it becomes eminent remains problematic.

For the purpose of detecting potential changes in the microcirculatory condition, there is a need for a monitor capable of reflecting the microcirculatory perfusion state.

Current systems for measuring gas employ a variety of measurement principles. In medical technology, predominantly electro-chemical sensors are used for measurement of gases that have diffused transcutaneously, i.e. that have diffused through the skin of a human or of an animal. Current sensors for measurement of transcutaneous O₂ or CO₂ are very sensitive and show good response times.

Typically the sensor houses a pH electrode, a reference electrode, an electrolyte solution, a membrane and a heating element.

The sensor may be fastened to the skin. The heating element warms the skin to a temperature sufficient to improve perfusion, in particular to a temperature above 40°C, more particular to a temperature from 42° to 45°C. The blood gas that diffuses through the stratum corneum by the warming of the skin passes across the sensor's semipermeable membrane and into an electrolyte solution in the sensor chamber.

A glass electrode measures the change of the pH-value. The electrode's output is converted into a signal indicative for the partial pressure of the blood gas. The signal may be read out or displayed.

Transcutaneous monitoring of the partial pressure of oxygen is widely used in neonates, since due to a thin epidermal layer the transcutaneous partial pressure of oxygen PtcO₂ reaches levels that are comparable to the arterial partial pressure of oxygen PaO₂. According to Tremper and Showmaker (Critical Care Medicine, Vol. 8, No. 10, pages 706-709, 1981) also for adult patients PtcO₂ has been reported to be a reliable trend monitor of PaO₂ in certain situations. To obtain a measurable reading when applied to the skin, the sensor must heat the skin to 42-45°C.

Heating of the skin increases blood flow to such an extent that oxygen levels increase and carbon dioxide levels decrease in the heated skin, a process which is called arterialization. When the skin is sufficiently heated and the skin and microcirculatory conditions permit, oxygen and carbon dioxide levels which correspond to arterial oxygen and carbon dioxide levels can be measured on the skin surface. This principle is applied in transcutaneous blood gas monitoring of patients.

Furthermore, the heat causes dermal hyperemia through dilation of arterioles, which is said to "arterialize the capillary blood" in terms of oxygen levels. However, PtcO₂ is a surface measure of heated skin tissue-oxygen tension and, therefore is a reflection of peripheral oxygen delivery. It has been shown that despite heating of the skin, sepsis severely impairs the skin microcirculation, and with it oxygen transport to the skin surface.

It is an object of the present invention to avoid the drawbacks of the state of the art and in particular to provide a device and a method for obtaining an indicator of the microcirculatory condition, in particular for indicating and/or predicting sepsis, which in particular allows a noninvasive and reliable measurement providing results within acceptable response times.

According to the invention this object is accomplished by a device for obtaining an indicator of a microcirculatory condition of a patient, which comprises at least one sensor for measuring data indicative of an arterial blood oxygen level, at least one sensor for measuring data indicative of a tissue oxygen level and a control unit for determining a measure of microcirculation on the basis of the tissue oxygen level and the arterial blood oxygen level.

Within this application "microcirculatory condition" means the amount of blood perfusion of the skin, in particular, measured as a difference between transcutaneously measured oxygen levels and arterial oxygen levels.

The sensor for measuring data indicative of an arterial blood oxygen level may be a sensor for indication of a general or systemic condition, in particular of a microcirculatory condition of a patient based on the measured data.

The sensors may detect one measured quantity or more measured quantities at a time or one after the other. The measured data may correspond to one or more parameters and/or to a temporal sequence of measured quantities.

The sensor for measuring data indicative of an arterial blood oxygen level and the sensor for measuring data indicative of a tissue oxygen level may be formed by the same sensor element, but working under different conditions. The sensor may for example be used for measuring data indicative of an arterial blood oxygen level at a first temperature and for measuring data indicative of a tissue oxygen level at second temperature. The sensor may be a heatable sensor, in particular for transcutaneous gas measurement, which may be suitable for being used in temperature cycles, such that during a first time period data indicative of an arterial blood oxygen level are acquired and during a second time period data indicative of a tissue oxygen level may be acquired. The time periods can run alternatingly.

A measure of microcirculation provides a statement on the degree of vascularization, blood flow and tissue perfusion and hence of the microcirculatory condition.

In particular the control unit is adapted for determining changes in tissue perfusion.

In particular the control unit has at least one input for receiving a measured or estimated first oxygen level value, for example a tissue oxygen level value, and at least one input for receiving a measured or estimated second oxygen level value, for example an arterial blood oxygen level value. Furthermore, the control unit may comprise at least one output interface for outputting an indicator of a microcirculatory condition of a patient based on the received inputs, for example a changed tissue perfusion condition based on a relative change of the received inputs

The control unit is particularly adapted for determining a measure of microcirculation in septic patients, where microcirculation is expected to be deteriorated due to decreased vascularization and blood flow in the superficial skin and other peripheral tissues.

The device uses the fact that the microcirculatory condition changes, when the relation between the arterial blood oxygen level and the tissue oxygen level changes. As long as the tissue perfusion is unaffected, the tissue oxygen level can be assumed to correspond to a measure of the arterial blood oxygen level. But when the tissue perfusion is impaired, the arterial blood oxygen level and tissue oxygen level are no longer corresponding parameters. Furthermore, the lack of correspondence can be used as a measure of the degree of the microcirculatory impairment.

A change of the tissue oxygen level can be caused by a change of the arterial blood oxygen level without any impact on the tissue perfusion. Thus, when the arterial blood oxygen level and tissue oxygen level are measured independently, it is possible to provide a reliable detection of a patient's microcirculatory condition.

A change of the microcirculatory condition, however, is not necessarily an indicator of a severe disturbance of blood flow, vascularization or tissue perfusion. Depending on the temporal course of the change it may anyway indicate a future likelihood of a problem. Hence, preferably, the control unit is adapted for prediction of a measure of microcirculation, preferably adapted for prediction of sepsis.

The sensor for measuring data indicative of an arterial blood oxygen level and/or the sensor for measuring data indicative of a tissue oxygen level may comprise a chemical sensor, an opto-chemical sensor, an optical sensor, an electrical sensor, an electro-chemical sensor, an opto-electrical sensor, a pressure sensor and/or a temperature sensor.

In an embodiment of the device the sensor for measuring data indicative of a tissue oxygen level is a first sensor element and the sensor for measuring data indicative of an arterial blood oxygen level is a second sensor element different from the first sensor element.

Two or more separate sensors allow a measurement of different data at the same time. Data measured at the same time or at least in a timely manner allow a reasonable comparison of the data.

There are several methods for determining an arterial blood oxygen level.

The second sensor element may be adapted to analyse a blood sample. A blood sample may be taken from the patient. The sample may be drawn from an artery or capillary vessel. The blood can also be drawn from an arterial catheter. Alternatively a continuous intra-arterial or intravascular measurement may be performed.

Blood samples may also be taken for calibrating a transcutaneous measurement as explained below.

A sensing unit with a second sensor element may comprise a microneedle. A microneedle may be applied to the tissue, preferably up to a distance of 0.2-1 mm from the skin surface. The microneedle may reach the capillary loops, such that oxygen reaches the needle tip.

The microneedle may be heated or non-heated. Preferable the body region around the measurement site is heated.

The microneedle may comprise an optical fibre and/or may use fluoroscopy.

The microneedle may comprise an optical fibre and a chemical sensor or an electrochemical probe, such as a Clarke probe for measuring oxygen concentration in a liquid.

The second sensor element may be adapted for transcutaneous measurement, preferably with a heating element, capable of heating the skin to a temperature above 40°C, preferably above 42°C, more preferably above 44°C, such that the transcutaneous measurement provides representative data for the arterial blood oxygen level in situations where the microcirculation is not impaired. This works best for neonates, wherein the absolute transcutaneous oxygen level generally corresponds to the arterial level. For older children and adults, having a thicker skin, there is less diffusion.

However, for an analysis absolute values are not necessary and a qualitative measurement would be sufficient.

The second sensor element may be adapted to use NIRS (near infrared spectroscopy) for estimation of the tissue oxygenation. The spectroscopy may be proceeded in tissue, hence as an invasive procedure.

In a preferred embodiment the second sensor element comprises a sensor adapted for measuring data indicative of the arterial blood oxygen level by optical detection of oxygen saturation, preferably by pulsoximetric detection.

The second sensor element preferably comprises an optical detector, which preferably uses two wavelengths, in particular a sensor for pulsoximetric detection.

Pulsoximetric detection provides a measure of haemoglobin saturated with oxygen as a percentage of the total haemoglobin. When measured peripherally on the body, the pulsoximetrically measured oxygen saturation is called SpO₂.

The sensor for pulsoximetric detection typically passes at least two wavelengths of light through the body part or tissue to a photodetector. It measures the changing absorbance at each of the wavelengths, allowing it to determine the absorbances due to the pulsing arterial blood alone, excluding venous blood, skin, bone, muscle and fat. Thus, the sensor is reading the peripheral oxygen saturation (SpO₂) which generally is correlated to the arterial oxygen saturation (SaO₂) from arterial blood gas analysis.

The reading of SpO₂, however, is not identical to the more desirable reading of SaO₂. The values both represent the arterial oxygen saturation of haemoglobin, yet are measured in a different manner. The relation to the arterial oxygen partial pressure (PaO₂) is described by the oxygen-haemoglobin dissociation curve, which may shift to the right or the left depending on a patient's conditions. Using the relationship between SaO₂ or SpO₂ and PaO₂ as described by the oxygen-haemoglobin dissociation curve, a PaO₂ estimate can be calculated from SpO₂. This estimate can be improved by input of other factors influencing the oxygen-haemoglobin dissociation curve, such as CO₂, temperature, 2-3 DPG, (fetal) haemoglobin and pH.

Hence, following a pulsoximetric detection an arterial partial pressure of oxygen is determined from measured SpO₂ values, preferably by using an oxygen-haemoglobin dissociation relationship.

Preferably the device comprises at least one further sensor, additional to the first and the second sensor, for measuring data indicative of a further parameter for determining the patient's condition, such as the temperature, the pH-value, carbon dioxide value of the blood and/or another blood parameter, in particular for correcting and/or calibrating of the arterial blood oxygen level and/or the tissue oxygen level.

The data may also be used to calibrate the sensor for pulsoximetric detection or to compensate deviations from a standard oxygen dissociation curve.

The further sensor adapted for measuring data indicative of a carbon dioxide level may make use of a transcutaneous measurement, preferably with a heated sensor. The carbon dioxide level may help to more precisely estimate PaO₂ using the correlation between the carbon dioxide levels and the oxygen dissociation curve.

The further sensor may be adapted for measurement of the temperature, preferably of the skin. The temperature may be used to correct the oxygen dissociation curve.

The further sensor may be adapted for measurement of a pH-value or 2,3-diphosoglycerat, another regulator for the binding affinity of haemoglobin. This measurement can take use of a blood sample, which is analysed.

The further sensor may also be adapted for measuring the foetal haemoglobin, when the device shall be used for neonates.

The first sensor, the second sensor and the further sensor may be arranged in a common housing.

The second sensor element may comprise a heating element. Stabilizing the temperature of the skin typically provides for reproducible data.

There are also several alternative methods for determining the tissue blood oxygen level. Hence there are several possible embodiments for the first sensor element.

The first sensor element may comprise a micro-needle which may be applied to the tissue. The needle may comprise a fibre optic for a spectroscopic measurement and/or a chemical sensor, which may be read out optically or electrically.

The first sensor element may be adapted to use NIRS (near infrared spectroscopy) for estimation of the tissue oxygenation. The spectroscopy may be proceeded in tissue, hence as an invasive procedure. Alternatively the spectroscopy may be proceeded non-invasively, for example in a sample of transcutaneous diffused gas in the vicinity of the skin.

Preferably the first sensor element comprises a sensor adapted for transcutaneous measurement, in particular for a heated transcutaneous measurement.

The first sensor element may comprise a sensor for transcutaneous oxygen measurement. The second sensor may also comprise a sensor for transcutaneous carbon dioxide measurement.

The first sensor element may comprise a pH electrode, a reference electrode, an electrolyte solution and a membrane. Optionally the second sensor element may comprise a heating element.

Preferably the device comprises at least one additional sensor in addition to first and second sensor. The additional sensor is adapted for measuring data indicative of a further parameter for determining the patient's condition, such as the temperature, the carbon dioxide level, the pH-value of the blood and/or another blood parameter, in particular for correcting the arterial blood oxygen level and/or the tissue oxygen level.

The additional sensor may be adapted for measuring data indicative of a carbon dioxide level.

The additional sensor may be adapted for measurement of the temperature, preferably of the skin.

The additional sensor may be adapted for measurement of a pH-value or 2,3-diphosoglycerate value. The measurement can take use of a blood sample, which is analysed.

The additional sensor may also be adapted for measuring the foetal haemoglobin, when the device shall be used for neonates.

The additional sensor may be adapted for measuring the heart rate and/or the heart rate variability.

The additional sensor may be adapted for measuring the pulse rate and/or the pulse rate variability.

The additional sensor may be adapted for measuring the electrical activity, such as ECG values, and/or muscle activity and/or blood flow and/or respiratory gas flow.

The device may comprise a heating element.

The sensor for measuring data indicative of an arterial blood oxygen level and/or the sensor for measuring data indicative of a tissue oxygen level, and/or if applicable, the further and/or the additional sensor, may be adapted for continuous and/or intermittent and/or alternating measurements.

A continuous measurement allows analysis of parameters over time. For parameters known to change slowly or for which a sudden change is not be relevant, a continuous measurement may mean that data are collected in predetermined time intervals, such as every few seconds or minutes.

In an advantageous embodiment of the device the sensor for measuring data indicative of an arterial blood oxygen level and the sensor for measuring data indicative of a tissue oxygen level, and preferably, if applicable, the additional sensor, are arranged in a common housing. The device may comprise a sensor head, which may be placed on the patient. The sensor head may house the sensor for measuring data indicative of an arterial blood oxygen level, the sensor for measuring data indicative of a tissue oxygen level and, if applicable, further sensors and/or additional sensors. By placing the sensor head all sensors are positioned with respect to the patient.

The sensors may be designed as combined sensors, using and/or sharing the same sensor elements, such as a temperature sensor.

Beneficially the sensor for measuring data indicative of an arterial blood oxygen level and/or the sensor for measuring data indicative of a tissue oxygen level are adapted to be placed anywhere on the skin or other peripheral tissue, for example on an earlobe or at a fingertip. The device may comprise a sensor head, preferably as described above, which is designed to form a clasp that grasps a finger or an earlobe.

In a preferred embodiment of the device the control unit is adapted for determining the difference between the tissue oxygen level and the arterial blood oxygen level, the ratio of the tissue oxygen level and the arterial blood oxygen level and/or an index based on the tissue oxygen level and the arterial blood oxygen level.

An index may be determined by a calculation based on the tissue oxygen level, the arterial blood oxygen level and optionally further measured or predetermined parameters.

The control unit may also be adapted for monitoring the tissue oxygen level, the arterial blood oxygen level, the difference, the ratio and/or the index over time and to determine a change over time.

In particular the control unit is adapted for receiving, collecting, storing and processing, particularly time-dependent, data.

In particular the control unit is adapted for determining changes in tissue perfusion on a predetermined and/or selectable timescale.

The control unit may be adapted for correcting the oxygen dissociation curve based on measurements of a further or additional sensor.

Furthermore the control unit may be adapted for extrapolation of time-dependent data and/or for prediction of, for example, a tissue perfusion state.

The control unit may be adapted to average and/or to filter measured and/or determined values and/or to determine a rolling average for a predetermined time interval.

The control unit may be adapted for comparing the tissue oxygen level, the arterial blood oxygen level, the difference, the ratio, the index and/or a deduced value with a respective nominal value.

In particular the control unit is adapted for determining the difference between the first and the second oxygen level, in particular the difference between the tissue oxygen level and the arterial blood oxygen level, the ratio of the first and the second oxygen level, in particular the ratio of the tissue oxygen level and the arterial blood oxygen level and/or an index based on the first and the second oxygen level, for example the tissue oxygen level and the arterial blood oxygen level.

The control unit may be adapted for receiving data from an input device, such as a control panel, a console or a data carrier reader.

In particular the control unit is adapted for receiving, storing and processing algorithms, correction parameter, nominal values and trigger values.

A correcting parameter may be used to process measured and/or determined data according to a rule. For example medication, vasotonic factors and the chemical drift of a sensor may be taken into account for analysing measured data.

The control unit may be adapted for indicating a disturbance of a sensor, a need for maintenance or a need for a new sensor calibration.

If the difference of a measured and/or determined value and a respective nominal value exceeds a predetermined trigger-value, the control unit may produce a respective output information on at least one output interface for submitting an indicator of a microcirculatory condition of a patient based on the received inputs. The output interface may for example display the existence of a health problem, may give a warning if a health problem is likely to occur or may provide a probability of a health problem to occur.

The device may provide a clear result based on a present measured and/or determined value. The device may provide a clear result based on a time development of a measured and/or determined value.

The control unit may be adapted to determine a difference, a ratio and/or an index on the basis of data measured during a time interval. Preferably the control unit may be adapted for a continuous determination on the basis of a rolling time interval.

Thus the device allows for a retrospective analysis of the data.

Changes due to sepsis are caused by slow processes. Thus, the result to be determined will take some time, typically a few minutes to a few hours. However, the device may provide an indicator of the microcirculatory condition and thereby an indicator of sepsis, faster than the conventional analysis of a blood sample, which takes one or more days.

In a preferential embodiment of the device the control unit is connected or connectable to an output device, such as a monitor or a display, preferably for displaying the measure of microcirculation and/or for displaying signals produced by the control unit.

The output device may be a part of the device.

The output device may be adapted to emit an acoustic and/or optical signal.

The output device may be adapted to display measured and/or determined values and/or a time dependent representation of measured and/or determined values.

The output device may be adapted to indicate a measure of the microcirculation. For example the output device may comprise a display being designed to comprise a three-level scale, wherein the first level indicates "no problem", the second level indicates "likelihood of a problem" and the third level indicates "attention: problem".

The output device may be adapted to display a measure for the quality of measured and/or determined values, such as an estimated measurement error or a standard deviation of an averaged value.

The output device may be adapted to display a measure for the quality of the measure of the microcirculation, such as a confidence index, which may be based on the quantity of data used. The more data used, the more trustworthy the result will be. Such a confidence index may be derived from a deviation of measurement data over a period of time; i.e. if the deviation is relatively low over a sustained period of time, the confidence index will be high.

In a beneficial embodiment of the device the control unit is adapted for indication of an arterial partial pressure of oxygen PaO₂ preferably estimated from SpO₂, and/or the control unit is adapted for indication of a transcutaneous partial pressure of oxygen PtcO₂.

The values measured by the described sensors may be displayed by the output devices. Thus, the user gets additional information and he can control the quality of the measure of microcirculation.

The device may be a stand-alone device to be placed at the bedside of a patient. The device may be a hook-up element for an existing system.

The device may be part of an existing system and may for example share sensors or an output device with an existing system.

The object of the invention is also accomplished by a method for obtaining an indicator of tissue perfusion, comprising the following steps. The indicator is preferably obtained with a device as described above.

An arterial blood oxygen level of a patient is provided and a tissue oxygen level of the patient, in particular a skin oxygen level, is provided.

A measure for the microcirculation is determined on the basis of the tissue oxygen level and the arterial blood oxygen level.

Preferably the measure for microcirculation is displayed in real-time.

The arterial blood oxygen level may be provided by a data-collection or may be measured, preferably by pulsoximetric measurement of SpO₂, wherein an arterial partial pressure of oxygen is calculated from the measured SpO₂ by use of the oxygen-haemoglobin dissociation relationship. Other measurement principles may be used as explained above.

The tissue oxygen level may be provided as a data collection or may be measured, preferably by a transcutaneous measurement. Other measurement principles may be used as explained above.

The measurements can be performed at the same time or alternating.

A measure for microcirculation may be determined on the basis of the tissue oxygen level and the arterial blood oxygen level.

Preferably the measure for microcirculation is displayed in real time, preferably basing on continuous and/or intermittent measurements.

A measure for microcirculation is determined for example by determining the difference between the tissue oxygen level and the arterial blood oxygen level, the ratio of the tissue oxygen level and the arterial blood oxygen level and/or an index based on the tissue oxygen level and the arterial blood oxygen level, preferably for a prediction of sepsis.

The object of the invention is also accomplished by a computer program for loading into a computer and/or for running on the computer, wherein the computer program is adapted for carrying out a method for obtaining an indicator of tissue perfusion as described above.

The computer program may be loaded and/or run on a control unit of a device as described above.

The computer program may be loaded and/or run on a central computer device of a medical clinic or practice or may be loaded and/or run on a measurement device for measuring an arterial blood oxygen level and/or a tissue oxygen level of the patient.

The invention is further explained with reference to preferred embodiments and the following drawings which show:
- Fig. 1:: A schematic representation of a first example of a device;
- Fig. 2:: a schematic view on a sensor head of a second example for a device;
- Fig. 3:: a schematic cross-sectional view of the sensor head of figure 2;
- Fig. 4:: a schematic representation of a third example of a device;
- Fig. 5:: a schematic cross-sectional view of the sensor head of the third example of a device;
- Fig. 6a:: a first schematic representation of an O₂ level;
- Fig. 6b:: a second schematic representation of an O₂ level.

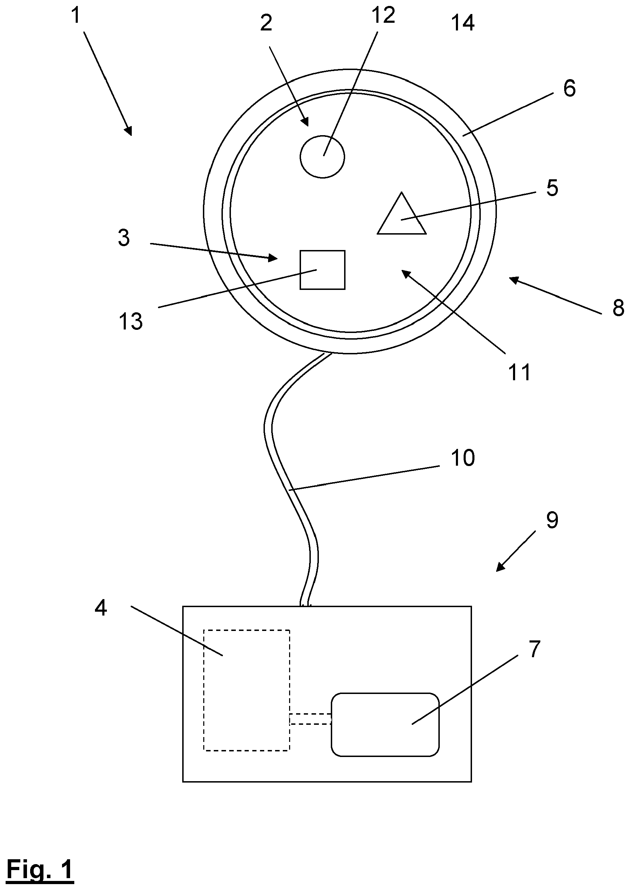

Figure 1 shows a schematic representation of a first example of a device 1 for for obtaining an indicator of a microcirculatory condition of a patient. The device 1 comprises a first sensor 3 for measuring data indicative of a tissue oxygen level and a second sensor 2 for measuring data indicative of an arterial blood oxygen level.

The second sensor 2 for measuring data indicative of an arterial blood oxygen level is a second sensor element 12 and the first sensor 3 for measuring data indicative of a tissue oxygen level is a first sensor element 13, in this example different from the first sensor element 12.

The device 1 comprises a further additional sensor 5 adapted for measuring data indicative of a carbon dioxide level, a pH-level and/or a temperature, in particular for correcting the pulsoximetric detection of the arterial blood oxygen level.

The first sensor element 13 and the second sensor element 12 are arranged in a common housing 6 forming a sensor head 8.

The device 1 comprises a control unit 4 for determining a measure of microcirculation, in particular changes in tissue perfusion, on the basis of the tissue oxygen level and the arterial blood oxygen level.

The device 1 comprises an additional sensor 5 for measuring data indicative of a further parameter, such as the temperature, the carbon dioxide level, the pH-value of the blood and/or another blood parameter, in particular for correcting the arterial blood oxygen level and/or the tissue oxygen level.

The sensor head 8 may be connected to a device base 9 by a cable 10. The control unit 4 may be arranged in the device base 9.

The control unit 4 is connected to an output device 7, such as a monitor or a display for displaying the measure of microcirculation. The output device 7 may also be arranged in the device base 9.

The sensor head 8 comprises a contact face 11 which is directable towards a measuring site. In this case, the measuring site is an area on the skin of a patient.

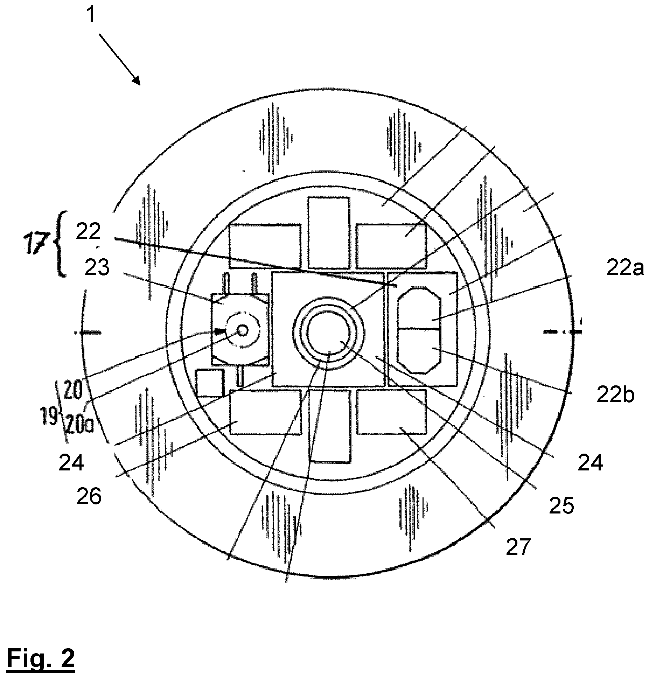

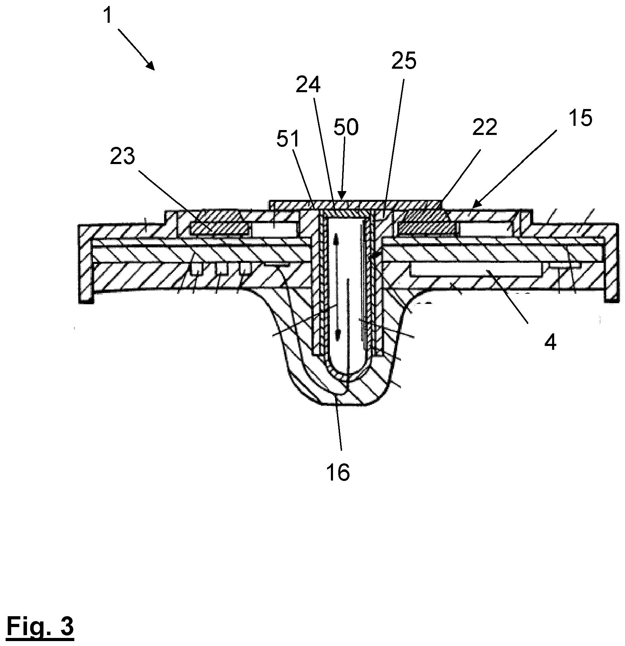

The device 1 shown in Figs. 2 and 3 allows a combined measurement of the arterial oxygen saturation (SpO₂) and of the transcutaneous O₂ partial pressure (PtCO₂).

For the measurement of the arterial oxygen saturation, the device 1 in this alternative embodiment according to Figure 2 comprises a second sensor 17 adapted for measuring the arterial blood oxygen level by pulsoximetric detection, hence a pulse oximetric measurement system which includes, among other things, a two-color light-emitting diode 22 (LED) as well as a photodetector 23. The two-color light-emitting diode 22 includes two light-emitting diodes 22a, 22b disposed closely next to one another and arranged in a common housing, with the one light-emitting diode 22a for example having a wavelength of approximately 660 µm (red) and the other light-emitting diode 22b for example having a wavelength of approximately 890 µm (infrared).

The device 1 has a surface 15 over which, in the embodiment shown, a membrane 50 is arranged and there between a thin layer of electrolyte 51. This membrane 50 is placed on the skin at a point of the human body which has a good blood flow, for example at a finger, at the forehead or at the earlobe. The light transmitted by the two light emitting diodes 22a, 22b radiates through the electrolyte 51 located above the light emitting diodes 22a, 22b and through the membrane 50 and is conducted into the body part, not shown, with a good blood flow and is scattered there and partly absorbed. The light reflected by the body part is measured using the photodetector 23. The signal measured by the photodetector 3 is supplied to a control unit 4.

The device 1 shown moreover includes a first sensor 19 adapted for transcutaneous measurement, hence an electrochemical measuring device, for the measurement of the transcutaneous oxygen partial pressure (PtcO₂ measurement), with this measuring device 19 preferably including a micro-pH electrode 24 as well as an Ag/AgCl reference electrode 25. The transcutaneous partial pressure of oxygen is, in this example, measured potentiometrically in that the pH of the thin layer of the electrolyte solution 51 is measured which is in communication with the skin via the hydrophobic membrane 50 which has good gas permeability. A change in the pO₂ value at the skin surface effects a pH change of the electrolyte solution. The pH is measured in that the potential is measured between the miniature pH electrode 24 and a reference electrode 25. The micro-pH electrode 24 is conductively connected via the electrical inner deflector 16 to the control unit 4.

The device 1 comprises a heating element 26 and a temperature sensor 27.

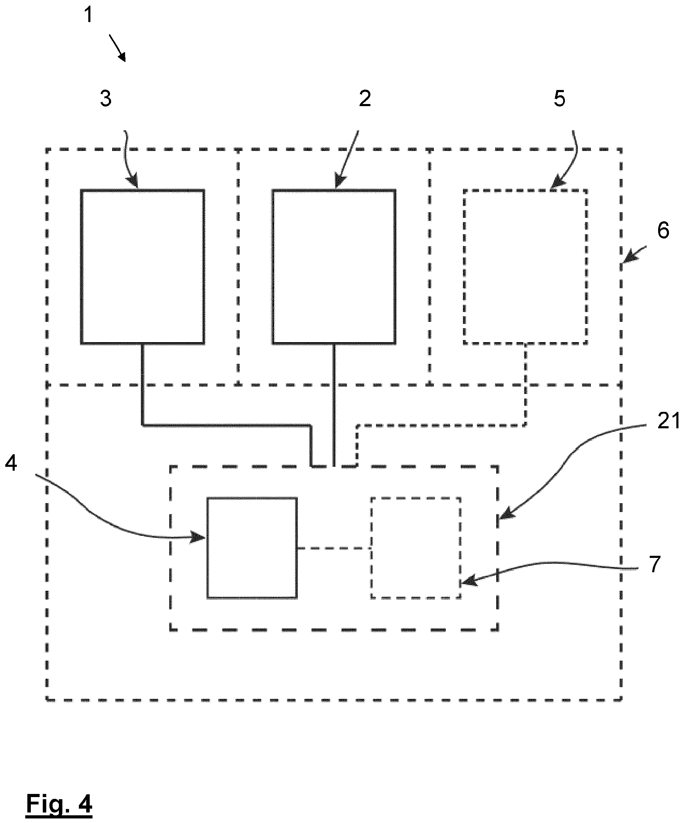

Figure 4 shows a schematic representation of a third example of a device 1 for for obtaining an indicator of microcirculatory condition of a patient.

A second sensor 2 for measuring data indicative of an arterial blood oxygen level, a so called blood oxygen sensor, a first sensor 3 for measuring data indicative of a tissue oxygen level, a so called tissue oxygen sensor, an additional sensor 5, a housing of a processor or control unit 4 and an output device 7 are arranged in a common housing 6.

Preferably the housing allows every combination, integration and separation of components.

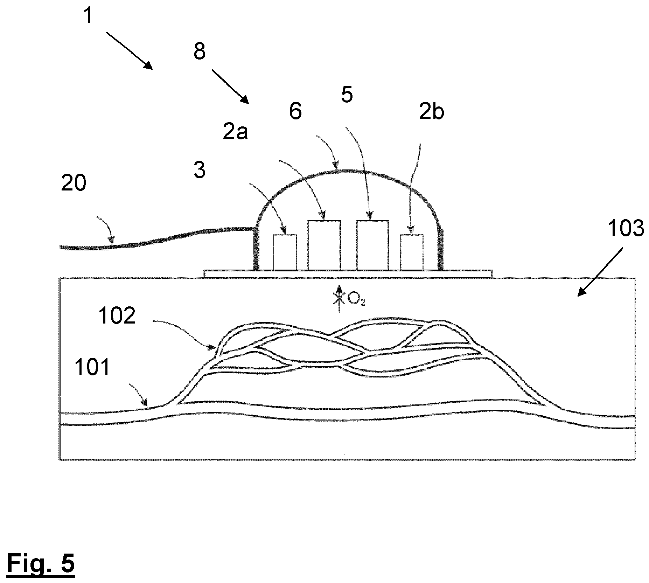

The second blood oxygen sensor 2 may be formed by a pulse oximeter and may comprise two parts 2a, 2b (see figure 5).

The first tissue oxygen sensor 3 may be a transcutaneous measurement device.

The additional sensor 5 may detect temperature, transcutaneous CO₂ and/or may comprise an input for external values.

The control unit 4 may provide a calculation of PaO₂ from a measured Sp0₂ value.

The output device 7 may comprise a display for showing a digital or analogue output.

Figure 5 shows a schematic cross-sectional view of the sensor head 8 of the third example of a device 1.

The sensor head 8 is placed in contact to the skin 103 of a patient.

A first sensor 3 for measuring data indicative of a tissue oxygen level, a so called tissue oxygen sensor, an additional sensor 5, a first part 2a and a second part 2b of a second blood oxygen sensor 2 are arranged in a common housing 6. A housing of a processor or control unit 4 and an output device 7 (see figure 4), not shown in this figure, may also be arranged in the housing 6.

The sensor head 8 is connected to an output connection 20, which may establish a connection to an external processor or controller and which may act as a power supply.

Accordingly, the device 1 for obtaining an indicator of microcirculatory condition of a patient, comprises at least one first sensor 3 for measuring data indicative of a tissue oxygen level, in particular a skin oxygen level, and at least one second sensor 2 for measuring data indicative of an arterial blood oxygen level and a control unit 4 (see figures 1 and 4) for determining changes in tissue perfusion.

The control unit 4 in particular has at least one input for receiving a measured or estimated arterial blood oxygen level value, at least one input for receiving a measured or estimated tissue oxygen level value, and at least one output interface for outputting an indicator of a microcirculatory condition of a patient based on the received inputs, not explicitly shown in the figures.

The skin 103 is permeated with arteries 101 and capillaries 102.

Blood supply in the arterioles and/or capillaries 102 may be impaired in a septic condition consequently significantly less oxygen diffuses to the skin surfaces, which may be detected by a transcutaneous measurement.

Blood supply in the skin arteries 101 remains unimpaired during sepsis. By using pulse oximetry the arterial oxygen saturation Sp0₂ can be measured and then the corresponding partial pressure PaO₂ may be calculated. The partial pressure PaO₂ may be compared to the transcutaneously measured oxygen tension PtcoO_{2.}

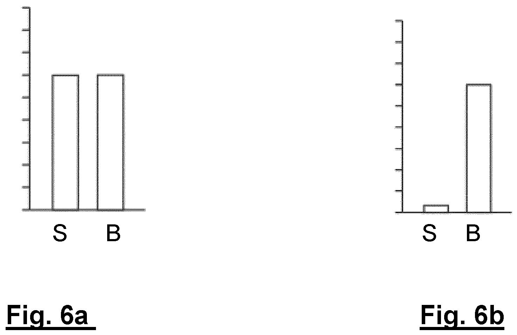

Figures 6a and 6b show representations of O₂ levels in the skin S and in the blood B.

Figures 6a shows that in a normal condition the oxygen level in the skin corresponds to the oxygen level in the blood.

To the contrary, as shown in Figure 6b, the oxygen level in the skin S is much lower than the oxygen level in the blood B in a septic condition.

## Claims

1. Device (1) for obtaining an indicator of microcirculatory condition of a patient, comprising
- at least one sensor (3) for measuring data indicative of a tissue oxygen level, in particular a skin oxygen level,
- at least one sensor (2, 2a, 2b) for measuring data indicative of an arterial blood oxygen level,
- a control unit (4)
for determining changes in tissue perfusion,
the control unit (4) in particular having at least one input for receiving a measured or estimated tissue oxygen level value, at least one input for receiving a measured or estimated arterial blood oxygen level value, and at least one output interface for outputting an indicator of a microcirculatory condition of a patient based on the received inputs.

2. Device according to claim 1, wherein
the sensor for measuring (3) data indicative of a tissue oxygen level is a first sensor element (13) and the sensor (2, 2a, 2b) for measuring data indicative of an arterial blood oxygen level is a second sensor element (12) different from the first sensor element (12).

3. Device according to claim 2, wherein
the second sensor element (12) comprises a pulsoximetric sensor (17) adapted for measuring the arterial blood oxygen level by optical detection of oxygen saturation.

4. Device according to claim 3, wherein
the device (1) comprises at least one further sensor (5, 14) adapted for measuring data indicative of a carbon dioxide level, a pH-level and/or a temperature, in particular for correcting a pulsoximetric detection of the arterial blood oxygen level.

5. Device according to claim 2, 3 or 4, wherein
the first sensor element (13) comprises a transcutaneous measurement sensor (19), in particular adapted for heated transcutaneous measurement.

6. Device according to one of the preceding claims, wherein the sensor (2, 2a, 2b) for measuring data indicative of an arterial blood oxygen level and/or the sensor (3) for measuring data indicative of a tissue oxygen level are adapted for continuous and/or intermittent and/or alternating measurements.

7. Device according to one of the preceding claims, wherein the sensor (2, 2a, 2b) for measuring data indicative of an arterial blood oxygen level and the sensor (3) for measuring data indicative of a tissue oxygen level are arranged in a common housing (6).

8. Device according to one of the preceding claims, wherein the sensor (2, 2a, 2b) for measuring data indicative of an arterial blood oxygen level and /or the sensor (3) for measuring data indicative of a tissue oxygen level are adapted to be placed on a part of the skin, in particular on an earlobe, at a fingertip, on a hand palm, on a foot and/or on the thorax.

9. Device according to one of the preceding claims, wherein the control unit (4) is adapted for determining an indicator of a microcirculatory condition of a patient based on
- the difference between a first and a second oxygen level, in particular the difference between the tissue oxygen level and the arterial blood oxygen level,
- the ratio of a first and a second oxygen level, in particular the ratio of the tissue oxygen level and the arterial blood oxygen level
and/or
- an index based on a first and a second oxygen level, in particular on the tissue oxygen level and the arterial blood oxygen level.

10. Device according to one of the preceding claims, wherein the control unit (4) is adapted for collecting, storing and processing time dependent data, in particular for determining changes in tissue perfusion on a predetermined and/or selectable timescale.

11. Device according to one of the preceding claims, wherein the control unit (4) is connected or connectable to an output device (7), such as a monitor or a display for displaying the measure of microcirculation.

12. Device according to one of the preceding claims, wherein the control unit (4) is adapted for indication of an arterial partial pressure of oxygen PaO₂ preferably based on SpO₂, and/or the control unit is adapted for indication of a transcutaneous partial pressure of oxygen PtcO₂.

13. Method for obtaining an indicator of tissue perfusion, comprising the steps of
- Providing an arterial blood oxygen level of a patient
- Providing a tissue oxygen level of the patient, in particular a skin oxygen level,
- determining a measure for microcirculation, preferably in septic patients,
on the basis of the tissue oxygen level and the arterial blood oxygen level.

14. Method according to claim 13 , comprising the steps of
- measuring an arterial blood oxygen level, preferably by optical detection, for pulsoximetric determination of an oxygen saturation,
- measuring a tissue oxygen level, preferably by a transcutaneous measurement.

15. Method according to claim 13 or 14, wherein the arterial blood oxygen level is provided by a pulsoximetric detection using two or more wavelengths.

16. Method according to claim 13 or 15, wherein further data are provided indicative of a carbon dioxide level, a pH-level and/or a temperature, in particular for correcting a pulsoximetric detection of the arterial blood oxygen level, preferably by measuring data indicative of a carbon dioxide level, a pH-level and/or a temperature.

17. Computer program for loading into a computer and/or for running on the computer, wherein the computer program is adapted for carrying out a method according to one of claims 13-16.
